# DEMANDE DE BREVET EUROPEEN

(11) **EP 0 617 937 A1**
(43) Date de publication de la demande: **05.10.1994**
(21) Numéro de dépôt: 94420058.3
(22) Date de dépôt: 18.02.1994
(51) Int. Cl.: A61F 7/00, A61H 15/00

(54) **Appareil délasseur par refroidissement notamment pour les jambes**

(30) Priorité: 19.02.1993 FR 9301913
(71) Demandeur: SEB S.A., F-69130 Ecully (FR)
(72) Inventeur: Bontoux, Daniel, F-69230 Saint Genis Laval (FR); Debourg, Jean-Pierre, F-69008 Lyon (FR); Paget, Monique, F-69300 Caluire (FR)

(57) **Abrégé**

- L'invention concerne un appareil délasseur, notamment pour les jambes.
- Selon un mode de réalisation préféré, l'appareil comprend un rouleau (30), en matériau hydrophile destiné à être appliqué sur une zone de la jambe (Ja) à délasser et à l'humidifier, un réservoir (5) de liquide comprenant au moins un orifice en regard du rouleau (30) laissant s'écouler le liquide et un système de ventilation (1) comprenant un moteur électrique (6) et un ventilateur (7) soufflant un jet d'air (4) sur la zone humidifiée de façon à l'assécher et à produire un abaissement superficiel de la température. L'appareil peut être alimenté par le secteur ou par des moyens autonomes : piles ou batteries.

## Description

L'invention concerne un appareil délasseur et plus particulièrement un appareil délasseur pour les jambes.

On connaît bien les phénomènes de "jambes lourdes" ressentis par exemple le soir après une longue journée de station debout.

Pour atténuer cette sensation, différents appareils sont proposés à cet effet et mis en vente dans le commerce. Ils permettent de procéder à des bains de pieds ou des bains bouillonnants. Il existe également des appareils de type vibrateurs. La mise en oeuvre de ces appareils est souvent laborieuse et l'efficacité de certains de ces appareils peut être mise en question.

On connaît également, de tout temps, des méthodes naturelles telles que les douches froides. On peut également soumettre le membre fatigué à un courant d'air frais.

L'expérience a montré, en effet, que la meilleure efficacité dans le domaine visé du délassement est obtenue par un refroidissement de la zone à délasser.

L'invention vise à pallier les inconvénients des dispositifs de l'art connu qui viennent d'être rappelés.

L'invention se fixe pour but la réalisation d'un appareil délasseur à la fois efficace, simple et pratique de mise en oeuvre.

Pour ce faire, l'appareil selon l'invention associe des moyens pour humidifier le membre à délasser, à un système de ventilation. L'humidification des membres, par exemple de la jambe à délasser, suivie d'un séchage provoque un refroidissement, de quelques degrés, mais très intense. De ce fait la sensation de lourdeur s'atténue.

L'invention a donc pour objet un appareil délasseur, notamment pour les jambes, comprenant des premiers moyens pour humidifier, à l'aide d'un liquide déterminé, et comprimer légèrement au moins une zone à délasser, caractérisé en ce qu'il comprend des deuxièmes moyens destinés à sécher ladite zone humidifiée de manière à obtenir une évaporation dudit liquide entraînant un abaissement superficiel de la température dans ladite zone.

L'invention présente donc l'avantage d'obtenir, comme il a été indiqué, tout à la fois un délassement efficace et d'être simple et pratique de mise en oeuvre.

En outre, l'appareil selon l'invention est, dans une variante préférée, du type portatif.

De préférence, les moyens d'humidification comportent un rouleau : pour obtenir ladite humidification, le rouleau est déplacé en une ou plusieurs fois le long du membre. Cette disposition est de nature à augmenter l'efficacité du délassement car il s'y ajoute un effet de massage.

L'invention sera mieux comprise et d'autres caractéristiques et avantages apparaîtront à la lecture de la description qui suit en regard des figures annexées et parmi lesquelles :
- la figure 1 illustre schématiquement la constitution et le fonctionnement d'un appareil délasseur selon l'invention ;
- la figure 2 illustre, en coupe, un exemple de réalisation pratique d'un appareil délasseur selon un premier mode de l'invention ;
- la figure 3 illustre, en coupe, un exemple de réalisation pratique d'un appareil délasseur selon un deuxième mode de l'invention ;
- la figure 4a illustre, en coupe, une variante de réalisation des moyens pour humidifier l'appareil conforme à l'invention ;
- la figure 4b est une vue agrandie du détail référencé par B à la figure 4a ;
- la figure 4c, est une vue en coupe suivant la ligne CC à la figure 4a ;
- la figure 5, illustre, en coupe, un exemple de réalisation pratique d'un appareil délasseur selon un troisième mode de l'invention ; et
- la figure 5a est une vue en coupe selon la ligne A-A à la figure 5.

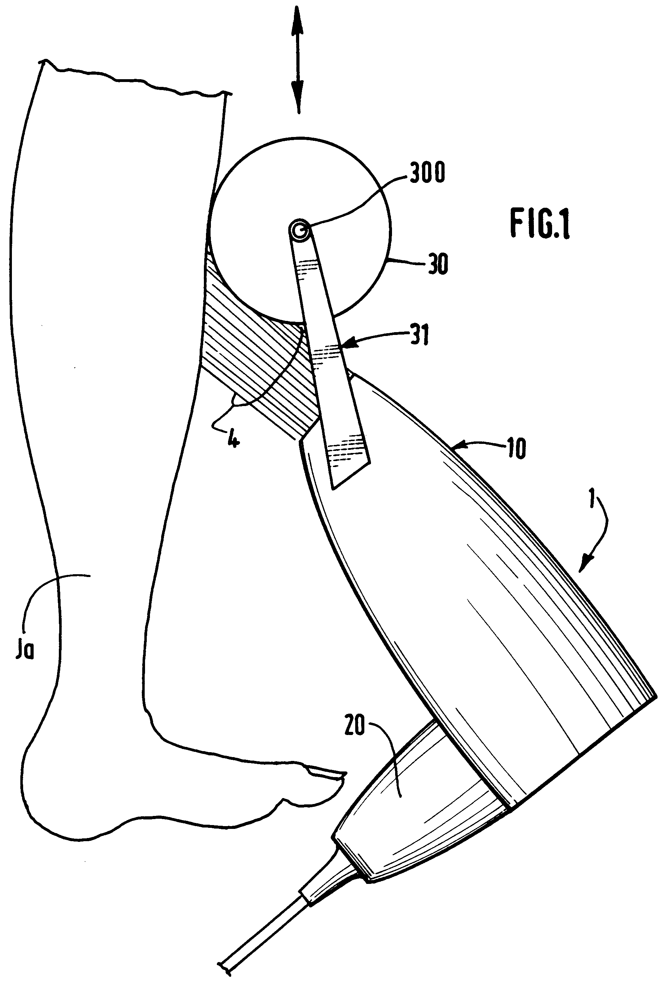

La figure 1 illustre schématiquement la constitution et le fonctionnement d'un appareil selon un mode de réalisation de l'invention.

Comme il a été précédemment indiqué, on associe deux moyens:
- des moyens d'humidification et de légère compression du membre à délasser ; et
- des moyens de ventilation et séchage simultanés ou quasi-simultanés de ce même membre.

C'est cette dernière opération qui permet le refroidissement précité et par là le délassement espéré.

On a représenté sur la figure 1 la jambe Ja d'une personne, jambe Ja que l'on désire délasser à l'aide de l'appareil selon l'invention. On appellera cette personne patient dans ce qui suit, ce par simple commodité.

Pour autant, l'appareil selon l'invention, bien qu'il puisse servir aussi en milieu médical, n'est pas destiné, en premier lieu, à ce type d'application mais, de façon avantageuse, à un usage dit "grand public".

L'appareil, illustré schématiquement par la figure 1, comprend un système de ventilation ou organe soufflant 1 muni d'un corps 10 et, de façon classique, de moyens de préhension 20 : poignée ou élément similaire, permettant de le tenir en main. Cet organe soufflant est destiné à générer de l'air froid 4 de préférence, c'est à dire à température ambiante, dirigé vers la jambe Ja à délasser.

A ce corps soufflant 10 est attaché un rouleau 30 par tous moyens appropriés. Sur la figure 1, on a représenté schématiquement ces moyens d'attache sous la forme d'un bras 31 supportant l'axe 300 du rouleau 30.

Le fonctionnement de cet appareil délasseur va maintenant être explicité.

On humidifie le rouleau 30 et on le maintient dans cet état. De façon préférentielle, celui-ci est constitué d'un matériau hydrophile, par exemple de la mousse hydrophile, au moins en périphérie.

On applique le rouleau 30 ainsi humidifié sur la peau du patient à l'endroit douloureux. L'organe souffle un jet d'air ambiant 4 au voisinage du rouleau 30 (à la base du rouleau 30, dans l'exemple illustré).

Lorsqu'on déplace l'appareil sur la peau du patient, le rouleau 30 tourne en provoquant un léger massage et en humidifiant la peau. Celle-ci est séchée par le courant d'air 4. Cette évaporation forcée de l'eau en superficie, comme il est bien connu par les lois de la physique, s'accompagne d'un refroidissement. La peau se refroidit donc fortement, une grande quantité de chaleur est évacuée provoquant un abaissement de la température superficielle de la zone traitée de quelques degrés: typiquement de 4 à 5°C. Ce phénomène provoque le resserrage des vaisseaux sanguins et lymphatiques, ce qui soulage le patient. La constriction ainsi provoquée aide au retour veineux et lymphatique et diminue la stase. Cet effet est augmenté si on utilise l'appareil de bas en haut pour la jambe, sens d'application que naturellement l'appareil autorise (ainsi que tout autre sens d'application).

Dans la version illustrée schématiquement par la figure 1, on suppose implicitement que le rouleau 30 est fixé rigidement, à l'aide du bras 31, au corps 10 de l'organe soufflant 1.

D'autres variantes de réalisation seront décrites ultérieurement. On peut cependant déjà indiquer que le bras pourrait être doté d'un certain degré de liberté, par exemple par pivotement limité autour d'un axe de fixation lié au corps 10, associé éventuellement à des moyens élastiques de rappel. Au lieu d'être couplé de façon fixe, l'ensemble rouleau 30 et bras 31 pourrait être pourvu de moyens permettant l'encliquement et le désencliquement sur et hors du corps 10 de l'organe soufflant.

Les moyens permettant de générer le jet d'air froid (air ambiant) sont en général constitués, comme il sera décrit ultérieurement avec plus de précision, par un moteur entraînant une turbine ou un ventilateur (non représenté sur la figure 1).

L'appareil délasseur selon l'invention n'étant pas pourvu de moyens de chauffage, il est à priori peu gourmand en énergie. On pourra donc l'alimenter, soit à partir du secteur (par exemple le secteur 220 V alternatif standard) soit avantageusement à partir d'une pile ou d'une batterie rechargeable, c'est-à-dire à partir de moyens autonomes.

Dans ce dernier cas, on améliore les aspects pratiques et la simplicité d'utilisation en supprimant le cordon électrique et en s'affranchissant de la nécessité de disposer d'une prise électrique à proximité. On obtient également une meilleure sécurité d'emploi sans recourir à des mesures d'isolation électrique renforcées.

Si on utilise une batterie rechargeable pour alimenter le générateur d'air dont est muni l'organe soufflant 4, on prévoit de façon avantageuse, dans une variante de réalisation (non représentée) un socle contenant un chargeur électrique apte à recevoir l'appareil délasseur selon l'invention et comportant des moyens de couplage électrique entre la batterie et le chargeur. De telles dispositions sont bien connues et couramment mises en oeuvre pour les petits appareils électroménagers rechargeables : aspirateur de table, etc... Il est inutile de les décrire plus avant.

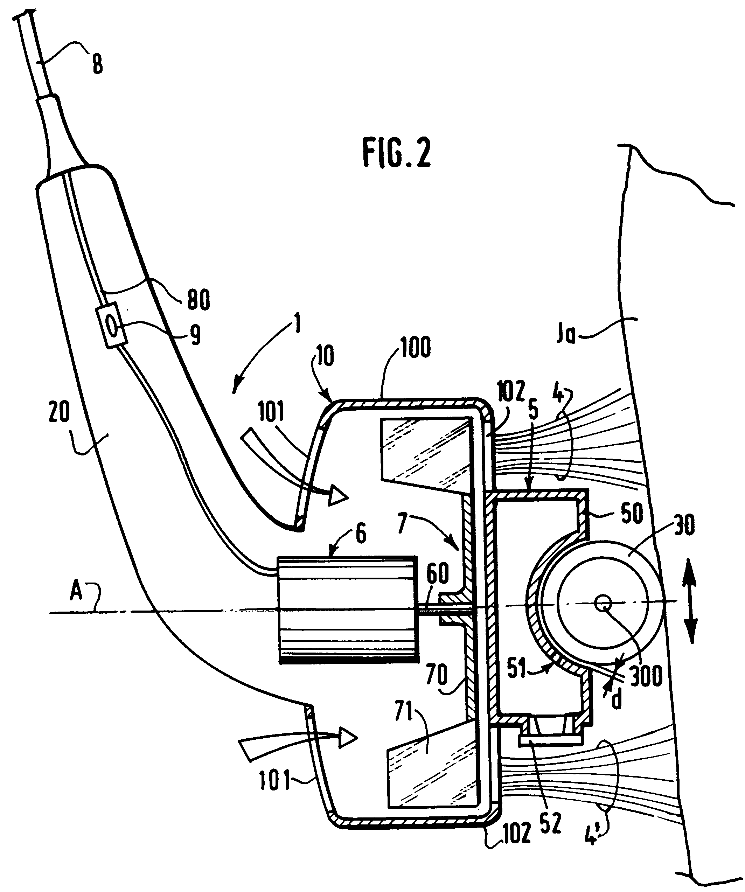

On va maintenant, par référence à la figure 2, décrire un exemple de réalisation pratique selon un premier mode de réalisation d'un appareil délasseur conforme à l'invention.

Les éléments identiques ou similaires à ceux de la figure 1 portent les mêmes références et ne seront redécrits qu'en tant que de besoin.

Cette figure représente, en coupe, les éléments principaux de l'appareil délasseur selon l'invention. On n'a pas représenté les moyens de fixation (fig. 1 : 31) du rouleau 30 sur le corps 10 de l'organe soufflant 1.

Conformément à ce mode de réalisation, on insère entre l'organe soufflant 1 et le rouleau 30 un réservoir 5 destiné à stocker le liquide dispensé sur le rouleau 30 pour l'humidifier. Il se compose d'une cavité 5 dont la paroi 50, dans une zone au regard du rouleau 30, épouse la forme du rouleau 30, c'est-à-dire une forme sensiblement cylindrique en creux. Cette portion de paroi est munie d'au moins un orifice 51 au regard du rouleau 50. De façon avantageuse, cette portion de paroi est munie d'une rangée d'orifices 51 parallèle à une génératrice du rouleau. Le réservoir comporte un bouchon 52 ou un élément analogue assurant l'étanchéité après remplissage de liquide. Ce dernier est en général de l'eau mais il peut y être adjoint toute solution appropriée.

Le rouleau 30 est réalisé, de façon préférentielle, en mousse hydrophile au moins dans une zone périphérique.

L'espace moyen ou jeu "d" entre la surface du rouleau et la paroi en regard est déterminé de façon à ce que celui-ci se remplisse, par capillarité, de l'eau ou plus généralement du liquide, contenu dans le réservoir 5. Les orifices 51 sont alors "noyés" et l'air ne peut plus entrer dans le réservoir 5 pour compenser la perte d'eau, ce qui arrête l'écoulement. La zone du rouleau 30 en regard de la paroi s'imbibe d'eau.

Quand le rouleau 30 tourne, le film d'eau compris dans l'espace d'épaisseur "d" est entraîné par celui-ci et se dépose sur la peau de la zone de la jambe Ja à traiter. L'air peut entrer à nouveau par le ou les orifices 51 et l'eau s'écouler à nouveau vers le rouleau 30, compensant ainsi la quantité déposée.

L'organe soufflant 1, comme précédemment, comprend un corps 10 et un organe de préhension 20, sous la forme d'une poignée, disposée (dans l'exemple illustré) à l'opposé du rouleau 30 et du réservoir de liquide 5.

Le corps 10 de l'organe soufflant dans l'exemple illustré présente une symétrie de révolution autour d'un axe ce qui fait qu'il est inscrit dans un cylindre, la paroi 100 de ce corps 10 formant la surface de ce cylindre. Dans la zone en regard de la poignée 20 que l'on appellera, par convention, zone arrière, la paroi 100 comporte un ou plusieurs orifices 101 laissant entrer l'air. Il en est de même dans la zone avant qui comprend au moins un orifice 102 destiné à expulser l'air. Les orifices 102 de la zone avant sont disposés, dans une variante préférée, de part et d'autre ou tout autour du réservoir 5. Celui-ci est disposé (toujours dans l'exemple illustré sur la figure 2) dans la zone centrale de la face avant.

Cette disposition améliore l'assèchement de la peau qui vient d'être humidifiée, ce quelle que soit la direction du mouvement et le sens de rotation du rouleau 30. Les jets d'air 4 et 4' sont dispensés de part et d'autre du rouleau 30, voire sous la forme d'une couronne, tout autour du rouleau 30.

L'organe soufflant 1 comprend, à l'intérieur du corps 10, un moteur 6 entraînant un ventilateur ou une turbine 7. Classiquement, celle-ci comprend un corps central 70 muni d'ailettes périphériques 71. Le moteur 6, centré sur l'axe de révolution précité, entraîne directement, par un axe 60, la turbine 7 dans l'exemple illustré. On peut naturellement avoir recours à d'autres variantes de réalisation et configuration connues, notamment en prévoyant un jeu d'engrenages. La variante de réalisation illustrée sur la figure 2 présent l'intérêt d'une très grande simplicité. Le coût en est minimisé d'autant.

On a supposé dans l'exemple illustré, que l'appareil était alimenté à partir du secteur standard par un câble 8. On a également représenté les fils internes 80 d'alimentation électrique du moteur 6, celui-ci étant mis sous et hors tension à l'aide d'un interrupteur classique 9.

Comme il a été indiqué, on peut naturellement alimenter le moteur 6 à l'aide d'une ou plusieurs piles ou de batteries rechargeables qui seraient logées avantageusement dans la poignée 20, comme il est d'usage classique pour les appareils électroménagers portatifs.

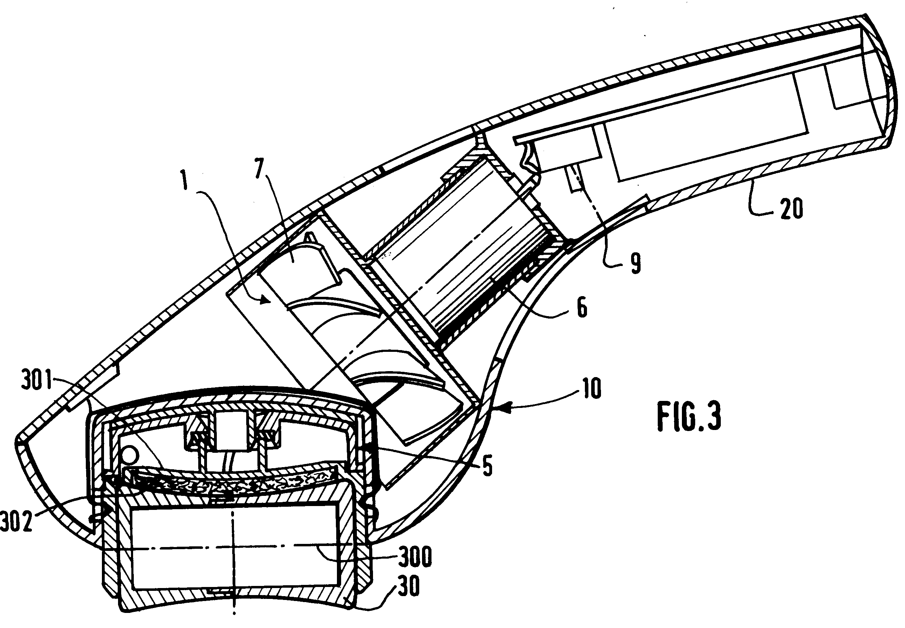

D'autres modes de réalisation sont décrits ci-dessous en référence avec les figures 3, 4a, 4b, 4c, 5 et 5a. Seules les différences entre ces modes de réalisation seront explicitées et les éléments communs ne seront pas repris.

En référence à la figure 3, dans cet exemple de réalisation, le rouleau 30 a une surface périphérique lisse.

Le réservoir 5 destiné à recevoir le liquide est également disposé entre le rouleau 30 et les deuxièmes moyens de séchage 1. L'espace laissé libre entre le rouleau 30 et le réservoir 5 a une épaisseur déterminée de manière à disposer, dans cet espace, un feutre 302 qui permet de transmettre par capillarité du liquide contenu dans le réservoir 5 sur la surface périphérique du rouleau 30.

Le réservoir 5 a une paroi 301, en regard du feutre 302, qui est percée localement de petits trous ou qui est poreuse.

Le feutre 302 peut être par exemple en fibres polyester. Le liquide provenant du réservoir 5 imbibe le feutre 302 qui frotte sur le rouleau 30 en l'humectant de façon uniforme.

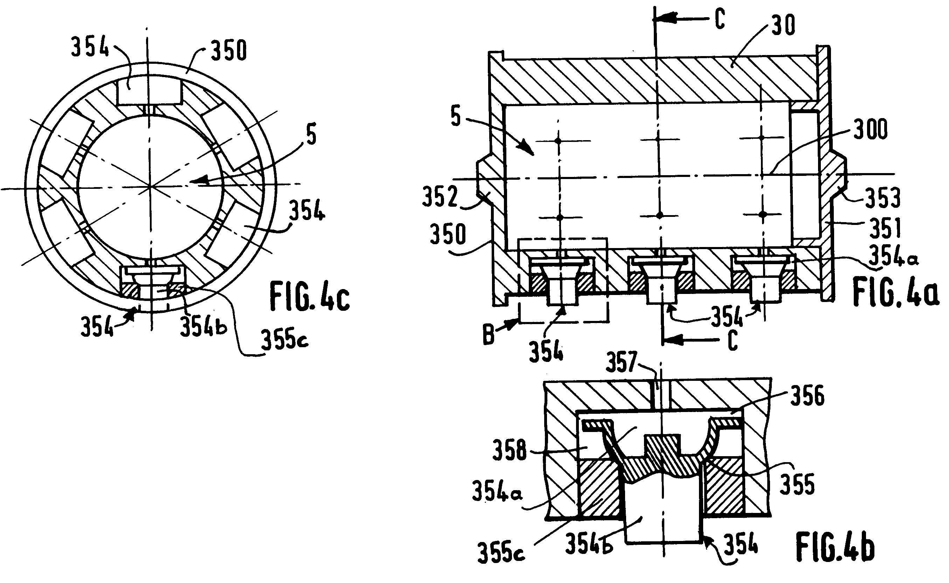

Selon un autre mode de réalisation illustré à la figure 4a, le rouleau 30 peut être un tube fermé à ses extrémités par des parois 350, 351, l'intérieur du rouleau constituant le réservoir 5 destiné à recevoir le liquide.

De préférence, l'une des parois 351 fait office de bouchon et permet de remplir le réservoir tout en assurant son étanchéité.

La paroi périphérique du tube comporte des éléments 354 formant clapet adaptés à laisser passer le liquide lorsque les éléments sont comprimés contre la zone à délasser.

La paroi périphérique du tube est de préférence une paroi épaisse de manière à loger des éléments 354 formant clapet dans des logements 354a ménagés dans l'épaisseur de ladite paroi.

Comme illustré à plus grande échelle à la figure 4b, les éléments 354 formant clapet sont semblables à des touches 354b analogues aux touches d'une calculatrice et montés librement mobiles dans les logements 354a entre deux positions de butée.

Ces touches 354b sont en élastomère et sont solidaires d'un clapet membrane 355 qui ferme de manière étanche l'orifice périphérique d'une bague 355c à travers lequel s'étend la touche 354b lorsque cette dernière est en saillie.

Lorsque le rouleau 30 vient en contact avec la zone à délasser, les touches 354b s'enfoncent et repoussent ainsi les clapets membranes associées 355.

Simultanément, un talon 356 solidaire de l'extrémité des clapets membranes 355 ferme l'arrivée du liquide en provenance de l'intérieur du tube et qui s'écoule par des orifices 357 situés sensiblement au droit des clapets membranes 355.

Une petite quantité de liquide contenu dans un espace 358, ménagé à l'intérieur de la paroi périphérique du rouleau 30, entre le talon 356 de l'élément 354 et l'orifice périphérique associé du tube, peut alors s'écouler dans l'orifice périphérique, autour de la touche 354b, pour venir humidifier la peau.

Comme illustré à la figure 4c, les éléments 354 et les orifices périphériques sont répartis de manière régulière sur la circonférence du rouleau 30 et forment des rangées alignées avec une génératrice du rouleau 30.

Dans cet exemple, le rouleau 30, qui sert de réservoir 5, possède six rangées de trois orifices périphériques, réparties sur sa circonférence.

De préférence, le rouleau 30 est amovible afin de faciliter son remplissage en liquide.

Des excroissances 352, 353 sont prévues sur les parois d'extrémité 350 et 351 de sorte que le rouleau 30 peut être tourillonné sur l'appareil.

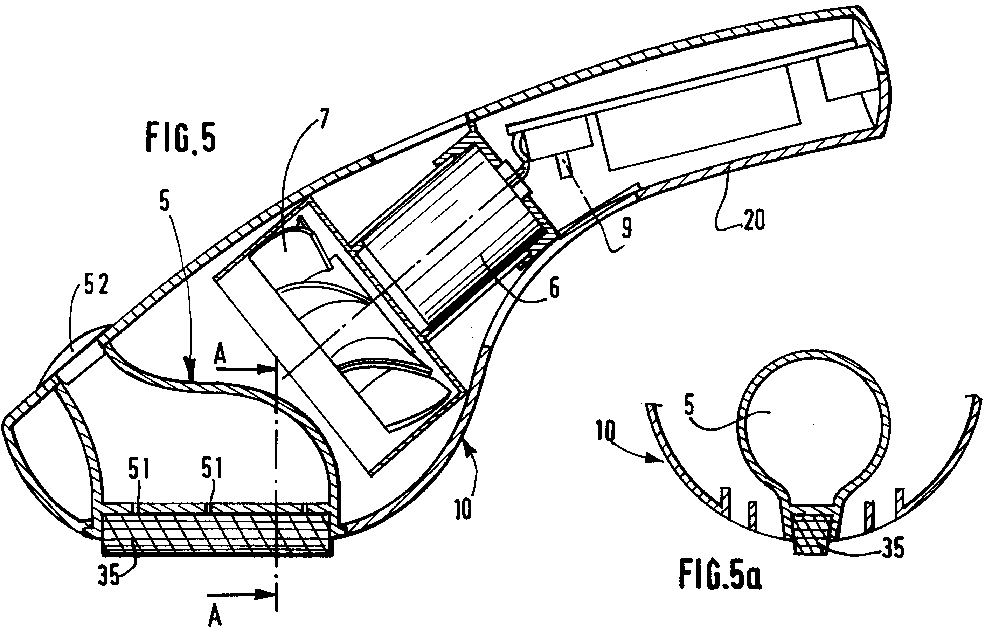

Dans une autre version de réalisation, illustrée aux figures 5 et 5a, les moyens d'humidification ne comprennent plus de rouleau 30 mais sont constitués d'un feutre 35 en matière hydrophile ou analogue.

Le liquide arrivant du réservoir 5 passe dans de petits orifices 51 pour humecter le feutre 35 par capillarité.

Le feutre 35 est déplacé sur la zone à délasser de manière à humidifier la peau et à la masser légèrement.

Les moyens de séchage 1, sont semblables à ceux des autres modes de réalisation et permettent d'obtenir un brusque refroidissement de la peau.

Naturellement, l'invention n'est pas limitée aux seules variantes de réalisation précisément décrites en relation, notamment, avec les figures 1 et 2.

En particulier, les moyens d'humidification du rouleau peuvent être différents d'un réservoir intégré tel qu'il a été décrit en relation avec la figure 2, même si cette disposition présente de grands avantages de simplicité et de régularité dans l'humidification.

On peut utiliser à titre d'exemple un simple spray de type manuel, un goutte-à-goutte, un système à mèche ou une pompe électrique puisant le liquide dans un réservoir annexe.

Enfin, bien que l'on n'ait pas besoin dans le cadre de l'invention de moyens de chauffage, on pourrait également, dans une variante de réalisation non représentée, utiliser comme organe soufflant, un sèche-cheveux classique. Il suffit alors de prévoir des moyens de couplage mécanique du sèche-cheveux à un ensemble comprenant au moins un rouleau et des moyens d'humidification de ce rouleau. Pour ce faire, on peut prévoir un support dont le profil est prévu pour s'emboîter sur l'embout soufflant du sèche-cheveux tout en laissant passer le flux d'air.

Les sèche-cheveux classiques sont en général pourvus de moyens de commande électrique multifonction, l'une de ces fonctions consistant à souffler de l'air froid (air ambiant).

Cette fonction pourra donc être utilisée avantageusement dans le cadre de l'invention pour assurer la fonction séchage, le sèche-cheveux pouvant par ailleurs continuer à être utilisé dans son usage d'origine après désaccouplement de l'ensemble d'organes constitué par un rouleau et des moyens d'humidification.

En dernier lieu, bien qu'un appareil portatif présente des avantages évidents dans son utilisation, rien ne s'oppose à ce que l'appareil selon l'invention soit relié à un bâti, vertical et horizontal, et se déplace le long de ce bâti, mu par des moyens mécaniques ou électrique (moteur par exemple). Il suffit alors que le patient, en position debout par exemple, place sa jambe parallèlement à la direction de déplacement de l'appareil le long du bâti et donc du déplacement du rouleau (le bâti étant supposé vertical).

## Revendications

1. Appareil délasseur, notamment pour les jambes (Ja), comprenant des premiers moyens pour humidifier, à l'aide d'un liquide déterminé, et comprimer légèrement au moins une zone à délasser, caractérisé en ce qu'il comprend des deuxièmes moyens (1) destinés à sécher ladite zone humidifiée de manière à obtenir une évaporation dudit liquide entraînant un abaissement superficiel de la température dans ladite zone.

2. Appareil selon la revendication 1, caractérisé en ce que lesdits deuxièmes moyens de séchage sont constitués par un système de ventilation (1).

3. Appareil selon la revendication 2, caractérisé en ce que ledit système de ventilation (1) comprend un moteur électrique (6) entraînant un ventilateur (7) générant un jet d'air (4, 4') à température ambiante et des moyens d'alimentation en énergie électrique (8, 80, 9).

4. Appareil selon la revendication 3, caractérisé en ce que les moyens d'alimentation en énergie électrique (8, 80, 9) sont dérivés du secteur.

5. Appareil selon la revendication 3, caractérisé en ce que les moyens d'alimentation en énergie électrique sont des moyens autonomes.

6. Appareil selon l'une des revendications 1 à 5, caractérisé en ce que lesdits premiers moyens sont constitués d'un feutre en matière hydrophile.

7. Appareil selon l'une des revendications 1 à 5, caractérisé en ce que lesdits premiers moyens comprennent un rouleau 30.

8. Appareil selon l'une quelconque des revendications 1 à 7, caractérisé en ce qu'il comprend un réservoir (5) destiné à recevoir ledit liquide.

9. Appareil selon les revendications 7 et 8, caractérisé en ce que ledit réservoir est disposé entre le rouleau (30) et lesdits deuxièmes moyens (1) de séchage, en ce que le réservoir (5) est muni d'au moins un orifice (51) en regard du rouleau (30) et en ce qu'un espace d'épaisseur (d) déterminée est laissé libre entre le rouleau 30 et le réservoir (5).

10. Appareil selon la revendication 9, caractérisé en ce que le rouleau (30) comporte au moins une zone périphérique en mousse hydrophile, ledit espace ayant une épaisseur (d) déterminée de manière à ce qu'un film de liquide se forme par capillarité par écoulement du liquide contenu dans le réservoir au travers de chacun des orifices (51) et s'étende sur ladite zone à humidifier lorsque le rouleau se déplace sur cette zone.

11. Appareil selon la revendication 9, caractérisé en ce que le rouleau (30) a une surface périphérique lisse, un feutre (302) étant disposé dans ledit espace, entre le rouleau et le réservoir, de manière à transmettre par capillarité, du liquide contenu dans le réservoir sur la surface périphérique du rouleau.

12. Appareil selon la revendication 9, caractérisé en ce que ledit réservoir (5) est muni de plusieurs orifices (51) disposés en regard d'une génératrice dudit rouleau (30).

13. Appareil selon les revendications 7 et 8, caractérisé en ce que le rouleau (30) est un tube fermé à ses extrémités par des parois (350, 351), l'intérieur dudit rouleau constituant le réservoir (5) destiné à recevoir le liquide, et en ce que la paroi périphérique dudit tube comporte des éléments formant clapet (354) adaptés à laisser passer le liquide lorsque lesdits éléments sont comprimés contre la zone à délasser.

14. Appareil selon la revendication 13, caractérisé en ce que le rouleau (30) est amovible.

15. Appareil selon l'une quelconque des revendications 8 à 14, caractérisé en ce que ledit réservoir (5) est muni d'un bouchon (52, 351) permettant son remplissage par ledit liquide et assurant l'étanchéité après remplissage.

16. Appareil selon l'une quelconque des revendications 1 à 15, caractérisé en ce qu'il est portatif et en ce qu'il est muni d'une poignée (20) permettant son déplacement dans l'espace.

17. Appareil selon l'une quelconque des revendications 1 à 16, caractérisé en ce que ledit liquide est de l'eau.
